# EUROPEAN PATENT APPLICATION

(11) **EP 4 524 125 A1**
(43) Date of publication of application: **19.03.2025**
(21) Application number: 22958169.9
(22) Date of filing: 09.09.2022
(51) Int. Cl.: C07C 59/08, C07C 53/08, C07C 59/06, C07C 211/10, C07C 215/10, C07C 229/06, C07F 3/02

(54) **HIGHLY ORIENTED METAL COMPLEX SALT**

(71) Applicant: Sea Water Chemical Institute, Inc., Kitakyushu-shi Fukuoka 807-1123 (JP)
(72) Inventor: MIYATA Shigeo, Kitakyushu City, Fukuoka 807-1123 (JP); HUNG-CUONG Dinh, Kitakyushu City, Fukuoka 807-1123 (JP)
(74) Representative: V.O.
(86) International application number: PCT/JP2022/033897
(87) International publication number: WO 2024/053091

(57) **Abstract**

[Problem] There is provided a low-cost material having a higher aspect ratio, which replaces the conventional high-aspect-ratio magnesium hydroxide requiring a large amount of monocarboxylic acid.

[Solution] There is provided a highly-oriented composite metal salt having a hexagonal Cd(OH)₂ type crystal structure and an X-ray diffraction intensity ratio (orientation H) of the (101) plane to the (001) plane of 60% or less, and being denoted by the following formula (1):

(Mg)₁₋ₓ(M²⁺)ₓ(OH)_{2-ny}A_{y} (1),

wherein "M²⁺" denotes at least one divalent metal other than Mg; "A" denotes at least one organic ligand; "x" denotes a number in a range of 0 ≤ x < 0.2, preferably 0 ≤ x < 0.1 and particularly preferably 0.01 ≤ x < 0.06; "y" denotes a number in a range of 0 < y < 0.05, preferably 0.0001 < y <0.02 and particularly preferably 0.001 < y <0.015; and "n" denotes an integer of from 1 to 4, preferably 1, or zero.

## Description

### Technical Field

The invention relates to a novel highly-oriented composite metal salt comprising Mg as a main component. More specifically, the invention relates to a novel highly-oriented composite metal salt, having an OH of Mg(OH)₂ partially substituted with an organic ligand, having very thin primary particles with a large lateral width, and being easily oriented in the lateral width direction.

### Background Art

Magnesium hydroxide often shows a plate-shaped crystal external form because it belongs to the hexagonal Cd(OH)₂ type structure whose crystal growth of the "c" axis direction (thickness) is poor compared with the "a" axis direction (lateral width). It is characterized in that, for example, it has a low specific gravity of 2.37, and is a non-toxic and basic material. It has a long history of uses as a stomach acid neutralizer and a laxative. However, the industrial uses of magnesium hydroxide, other than for flue gas desulfurization, are few with low demand.

On the other hand, the present inventors have developed magnesium hydroxide (Product name: KISUMA^{®} 5) being close to a monodisperse with nearly no secondary aggregation (primary particle size = secondary particle size) and having a primary particle (single crystal) with a lateral width of about 0.8 to 1 µm and a thickness of about 0.2 µm (Patent Document 1), so that a large market for halogen-free flame retardants of resins has opened up. Magnesium hydroxides prior to this development had a primary particle lateral width of about 0.2 µm or lower, and a strong aggregability so that secondary particles (which are produced by aggregation of the primary particles and are measured by particle size distribution) were as large as 5 µm or more.

In order to further expand the applications of magnesium hydroxide-based compounds, the present inventors have developed a high-aspect-ratio magnesium hydroxide-based solid solution, having a hexagonal plate crystal with an average lateral width of 1 to 10 µm, a thickness of 0.01 to 0.5 µm and an aspect ratio of 10 or more and being denoted by the following formula (2):

Mg₁₋ₓM²⁺ₓ(OH)₂ (2),

wherein M²⁺ denotes at least one divalent metal selected from the group consisting of Mn²⁺, Fe²⁺, Co²⁺, Ni²⁺, Cu²⁺ and Zn²⁺; and "x" denotes a number in a range of 0.01 ≤ x < 0.5.

This solid solution is produced by a method comprising steps of: sintering a hydroxide denoted by formula (2) to form an oxide, and subjecting the oxide to hydration reaction in the presence of a monocarboxylic acid and/or an oxymonocarboxylic acid in an aqueous medium. Achievement of the high aspect ratio has opened up novel applications such as a resin reinforcing agent (Patent Document 2).

As a result of further advancing the research about the high-aspect-ratio magnesium hydroxides, the present inventors have developed a high-aspect-ratio magnesium hydroxide having a lateral width of 0.5 µm or more, a thickness of 0.2 µm or lower and an aspect ratio of 10 or more, by the method comprising steps: (A) adding an alkali to a mixed aqueous solution of a water-soluble magnesium salt and an alkali metal salt and/or ammonium salt of a monovalent organic acid to cause coprecipitation product; or (B) adding an alkali to an aqueous solution of a water-soluble magnesium salt to cause coprecipitation and then adding an alkali metal salt and/or ammonium salt of a monovalent organic acid thereto; and (C) hydrothermally treating a resulting slurry of coprecipitation product at 100°C or more (Patent Document 3).

The present inventors furthermore have developed a method for producing a high-aspect-ratio magnesium hydroxide-based solid solution denoted by formula (2) at reduced cost. The method comprises steps of: reacting a mixed aqueous solution of a water-soluble Mg salt (A) and a water-soluble divalent metal salt (M²⁺) or metal complex thereof (B) with an alkali aqueous solution (C) such as sodium hydroxide in the presence or absence of monocarboxylic acid ions to obtain a coprecipitation product (D); and then heating and aging the coprecipitation product (D) in the presence of at least one chloride (E) selected from the group consisting of sodium chloride, potassium chloride, ammonium chloride, magnesium chloride and calcium chloride at 60 to 300°C (Patent Document 4).

Meanwhile, with the purpose of developing fine particles of magnesium hydroxide, there is provided a magnesium hydroxide-based solid solution having an average secondary particle size of not more than 300 nm, wherein the solid solution has a hydroxyl group OH partially substituted with a monovalent organic acid, and is denoted by the following formula (3):

Mg (OH)₂₋ₓRₓ (3),

wherein R denotes a monovalent organic acid and "x" denotes a number in a range of 0 < x < 1). The fine particles of the solid solution are produced by the following method (A) or (B). The method (A) comprises steps of: subjecting a mixed aqueous solution of a water-soluble magnesium salt and a monovalent organic acid or a salt thereof to addition of an alkali in an amount substantially equivalent to Mg to obtain a coprecipitation product and then hydrothermally treating the coprecipitation product. The method (B) comprises steps of: subjecting an aqueous solution of a water-soluble magnesium salt to addition of an aqueous solution of an alkali to cause coprecipitation; adding a monovalent organic acid or a salt thereof to the resulting magnesium hydroxide; and then applying hydrothermally treatment thereto (Patent Document 5).

### Prior Art Document

### Patent Document

Patent Document 1: JPS52-11579A
Patent Document 2: JPH08-259235A
Patent Document 3: WO 2012/050222A1
Patent Document 4: JP 2020-152626A
Patent Document 5: WO 2016/031803A1

### Summary of the Invention

### Problem to be Solved by the Invention

The reinforceability for a resin becomes higher as the aspect ratio becomes higher, thereby expanding the applications of magnesium hydroxide-based compounds. However, the aspect ratio of conventional magnesium hydroxide-based compounds is 50 or lower at most. Accordingly, the first object of the invention is to provide a high-aspect-ratio magnesium hydroxide-based compound having an aspect ratio of more than 50.

In the conventional method of producing a high-aspect-ratio magnesium hydroxide, the aspect ratio tends to become higher as an amount of a monovalent organic acid or a salt thereof becomes higher relative to Mg, so that it is necessary to use the monovalent organic acid or the salt thereof in an amount as large as 1 to 1.5 moles per mole of Mg. Since the conventional method requires a large amount of monovalent organic acid which is more expensive than an Mg raw material, the cost for raw materials become high. This is further coupled with the difficulty of separating the monovalent organic acid from water. At the same time, costly wastewater treatment facilities are necessary since wastewater contains a large amount of monovalent organic acid. In addition, the running costs thereof are necessary. Consequently, the cost for production becomes high. Accordingly, the second object of the invention is to provide a low-cost production method.

### Solution to the Problem

As a result of earnest investigations to achieve the objects, the inventors have succeeded in developing a highly-oriented composite metal salt having a hexagonal Cd(OH)₂ type crystal structure and an X-ray diffraction intensity ratio (orientation H) of the (101) plane to the (001) plane of 60% or less, and being denoted by the following formula (1):

(Mg)₁₋ₓ(M²⁺)ₓ(OH)_{2-ny}A_{y} (1),

wherein "M²⁺" denotes at least one divalent metal other than Mg; "A" denotes at least one organic ligand; "x" denotes a number in a range of 0 ≤ x < 0.2, preferably 0 ≤ x < 0.1 and particularly preferably 0.01 ≤ x < 0.06; and "y" denotes a number in a range of 0 < y < 0.05, preferably 0.0001 < y <0.02 and particularly preferably 0.001 < y <0.015; and "n" denotes an integer of from 1 to 4, preferably 1, or zero.

The X-ray diffraction intensity ratio (orientation H) is, for example, 60% or less, preferably 30% or less, more preferably 3% or less, and particularly preferably 1% or less.

In one of the embodiments, "n" denotes an integer in the range of 1 to 4, and the X-ray diffraction intensity ratio (orientation H) is 60% or less, preferably 30% or less, more preferably 3% or less, and particularly preferably 1% or less.

**In** one of the embodiments, "n" denotes zero.

The inventors have also succeeded in developing a method for producing the highly-oriented composite metal salt, the method comprising steps of:
subjecting an aqueous solution of one or more divalent metals to addition of less than 10% by mol, preferably 5% by mol or less of an organic ligand relative to a total molar amount of the divalent metals to obtain a mixture, wherein the solution comprises magnesium;
reacting the mixture with the 0.95 equivalent or less of an alkali relative to a total equivalent of the divalent metals to obtain a coprecipitated product; and
hydrothermally treating the coprecipitated product at 100°C or higher.

A method for producing the highly-oriented composite metal salt comprises steps of:
subjecting an aqueous solution of a water-soluble magnesium salt to addition of about 0.01 moles of an organic ligand capable of forming a metal complex per mole of Mg;
supplying 0.95 equivalent or lower of an alkali, preferably 0.9 equivalent or lower of an alkali, relative to Mg thereto to obtain a coprecipitated product; and
hydrothermally treating the coprecipitated product at 100°C or higher.

As a result, a highly-oriented (high aspect ratio) magnesium hydroxide can be produced at reduced production cost. The required amount of organic ligand may be a very small amount, equivalent to about 1/100th of the conventionally required amount of monovalent organic acid or a salt thereof. Amounts more than or less than the above required amount will conversely drastically reduce the orientation (aspect ratio). This is a new discovery completely unexpected from the conventional concept that an aspect ratio becomes higher as the amount of monovalent organic acid is increased.

According to the invention, a second important production condition is the added equivalent of the alkali, that is, 0.95 equivalent or lower, preferably 0.9 equivalent or lower, and particularly preferably 0.85 equivalent or lower and 0.6 equivalent or higher, relative to Mg. By limiting the amount of alkali to less than 1 equivalent, the organic ligand can function.

### Effect of the Invention

According to the invention, the high-oriented composite metal salt allows the primary particles (crystallites) to become thinner (about 5 nm as a minimum value), conversely the lateral width to become greatly wider (about 40 µm as a maximum value), and aggregation to be reduced in comparison with the conventional art of utilizing a monovalent organic acid or a salt thereof. Thus, according to the invention, the composite metal salt becomes very highly-oriented and therefore has a higher aspect ratio (a maximum of about 200 in contrast to a conventional maximum of about 40). Accordingly, the reinforcing effect for a resin is higher than that of the conventional product, and the resin can be strengthened with a lesser amount, thereby contributing to a further weight reduction of automobiles. Furthermore, according to the invention, the highly-oriented composite metal salt widens the scope of its application to the other automobile parts such as door trimmings, bumpers and instrumentation panels.

According to the invention, examples of the applications of the highly-oriented composite metal salt can include, in addition to the application of automobile resins, flame retardants, smoke-suppressing agents, thermally conducting agents, gas-barrier materials, oxygen absorbents and biodegradation promoters. They can further include an anti-rust agent for paint, a dyeing agent for fiber and a flame retardant for paper. Moreover, according to the invention, taking advantage of characteristics such as excellent lubricity and pearl tone glossiness, the highly-oriented composite metal salt can also be utilized as lubricating powder for cosmetics, instead of mica or talc.

According to the invention, the amount of organic ligand required in the production of the highly-oriented composite metal salt is as extremely small as about 0.01 moles per mole of Mg, so that expenses for raw materials can be greatly reduced in comparison with those for the conventional art. Further, steps and facilities to collect organic matter can be omitted, thereby further reducing the cost.

### Brief Description of Drawings

Fig. 1 shows XRD patterns of the products obtained in Example 1, Example 4 and Comparative Example 5.
Fig. 2 shows SEM photographs obtained in Example 1 and Example 4.

### Mode for Carrying Out the Invention

According to the invention, a novel synthesis is discovered in which an organic ligand capable of forming a metal complex is used instead of a conventional monovalent carboxylic acid; an alkali is used for the coprecipitation reaction in an amount 0.95 equivalent or less, preferably 0.9 equivalent or less, particularly preferably 0.85 equivalent or less and 0.6 equivalent or more, relative to Mg; and after the coprecipitation reaction, hydrothermal treatment is carried out at 100°C or higher, preferably 120°C or higher, and particularly preferably 180°C or higher.

The invention has been completed as a result of the earnest investigations based on the following concept. By creating conditions in which an Mg ion and an organic ligand would easily form a complex, there can be added two new functions which the conventional art does not provide. The first function is that, because the organic ligand bonds with the Mg ion at two or more sites, the growth of the thickness direction of primary particles can be effectively suppressed with a small amount of the organic ligand, in comparison with the conventional monovalent organic acid which can bond only at one site. The second function is that, because the complex with Mg is water-soluble to a certain extent as the feature of a metal complex, improvement of solubility can be expected, and as a result, the growth of primary particles is promoted and aggregation is reduced, thereby improving dispersibility.

According to the invention, the highly-oriented composite metal salt denoted by formula (1) has the following characteristics.
(1) The powder X-ray diffraction pattern has the same hexagonal Cd(OH)₂ type crystal structure as that of magnesium hydroxide. Although the OH group of Mg(OH)₂ is partially substituted with an organic ligand, the crystal structure is the same as that of Mg(OH)₂.
(2) The X-ray diffraction intensity ratio H (orientation) of the (101) plane to the (001) plane is 60% or less, preferably 30% or less, and particularly preferably 3% or less.

This means that as the orientation H becomes higher, the aspect ratio of primary particles becomes higher with less aggregation. As a result of repeating Examples of Patent Document 5, the Examples relating to the same solid solution as that in the invention, the orientation H of the glycolic acid solid solution (in Example 2) and the orientation H of lactic acid solid solution (in Example 3) were 143% and 147%, respectively, obviously differing from the highly-oriented composite metal salt in accordance with the present invention.
(3) The average lateral width of the primary particles is 0.21 µm or more, more preferably 2 µm or more, still more preferably from 3 to 50 µm, and further preferably from 3 to 20 µm. Alternatively, the average lateral width of the primary particles is more preferably from 4 to 50 µm, still more preferably from 4 to 20 µm, and particularly preferably from 4 to 10 µm.
(4) The average thickness of the primary particles is in the range of from 1 to 100 nm, preferably from 5 to 100 nm, more preferably from 5 to 70 nm, still more preferably from 5 to 60 nm, and particularly preferably from 5 to 50 nm. Alternatively, the average thickness of the primary particles is preferably from 10 to 100 nm, more preferably from 10 to 70 nm, still more preferably from 10 to 60 nm, and particularly preferably from 10 to 50 nm.
(5) The aspect ratio, which is the ratio of the lateral width to the thickness of primary particles, is 20 or more, preferably 50 or more, and particularly preferably 100 or more.
(6) The average secondary particle size is substantially the same as or slightly larger than the primary particle size, so that there is little secondary aggregation.
(7) When the primary particles are of 2 µm or more, the lubricity (low dynamic friction) and glossiness (pearl tone) are excellent.
(8) The endothermic decomposition peak temperature differs from that of magnesium hydroxide, and has a maximum change of about 20°C.

According to the invention, the organic ligand constituting the highly-oriented composite metal salt preferably has one carboxyl group or one sulfo group. It is preferably a bidentate ligand which is slightly larger than the OH group. Preferable Examples of the organic ligand include (1) oxycarboxylic acids (also hydroxycarboxylic acids) such as glycolic acid, lactic acid, glyceric acid, hydroxybutyric acid, pantoic acid, quinic acid, salicylic acid, vanillic acid, syringic acid, orsellinic acid, hydroxybenzoic acid, vanillic acid, gallic acid, mandelic acid and benzilic acid, and more preferably such as glycolic acid, lactic acid, glyceric acid, hydroxybutyric acid, hydroxybenzoic acid, vanillic acid, gallic acid and mandelic acid; (2) amines such as ethylenediamine, hexamethylenediamine, diethanolamine, triethanolamine, putrescine, cadaverine, ethambutol, phenylenediamine, toluidine, piperazine, imidazole, pyridazine, pyrimidine, pyrazine, oxazole and thiazole, and preferably such as ethylenediamine, hexamethylenediamine, diethanolamine, triethanolamine, cadaverine, toluidine, piperazine, imidazole, pyridazine, pyrimidine, pyrazine, oxazole and thiazole; (3) aminocarboxylic acids such as glycine, tryptophan, histidine, glutamic acid, aspartic acid, proline, 2-aminobenzene-carboxylic acid and 4-aminobenzene-carboxylic acid; (4) sulfonic acids such as aminoethanesulfonic acid, 2-aminobenzenesulfonic acid and 4-aminobenzenesulfonic acid; (5) polyhydric alcohols such as ethylene glycol, propylene glycol and glycerin; and (6) polyphenols such as anthocyanin and catechin.

Particularly preferable examples of the organic ligand are the aforementioned (1) and (2).

The "x" of organic ligand is in the range of 0 ≤ x <0.2, preferably 0 ≤ x < 0.1, and particularly preferably 0.01 ≤ x <0.06.

The "y" of organic ligand is in the range of 0 < y <0.05, preferably 0.0001 < y < 0.02, and particularly preferably 0.001 < y < 0.015.

The "n" denotes an integer in the range of from 1 to 4, and is preferably 1 or zero.

According to the invention, examples of the highly-oriented composite metal salt include, in addition to pure magnesium hydroxide, a solid solution in which an Mg of Mg(OH)₂ is partially substituted with at least one of another divalent metal M²⁺, preferably Ca²⁺, Mn²⁺, Fe²⁺, Co²⁺, Ni²⁺, Cu²⁺ and Zn²⁺, and particularly preferably Ca²⁺, Fe²⁺, Ni²⁺ and Zn²⁺. The "x" of the solid solution of M²⁺ is in the range of 0 ≤ x <0.3, preferably 0 ≤ x ≤ 0.2, and particularly preferably 0 ≤ x ≤ 0.1. Ca²⁺ strengthens the basicity, Ni²⁺ and Zn²⁺ improve the flame retardancy by catalytic activity of dehydrogenation, and Ni²⁺ improves acid resistance. Further, Mn²⁺, Fe²⁺ and Co²⁺ have oxygen absorbency, as well as oxidative degradation activity of resin, and thus can be mixed into foodstuff packaging resin film to be used as an oxygen absorbing material and/or a biodegradation promoter. Fe²⁺ can further be used as a therapeutic agent for iron-deficiency anemia.

According to the invention, examples of the appropriate applications of the highly-oriented composite metal salt include, in addition to the above-mentioned applications, mechanical reinforcing agents of resins such as polypropylene, flame retardants, thermally conducting modifiers (heat dissipation materials), gas-barrier materials, smoke-suppressing agents, fillers for artificial marbles, lubricating powder base materials for cosmetics (functional substitute for talc or mica), base materials for pearl pigments (surface coating by fine particles of titanium oxide, iron oxide, etc.) and therapeutic agents for iron-deficiency anemia.

According to the invention, the highly-oriented composite metal salt sintered at 400 to 1000°C is thin in thickness, highly-oriented and excellent in dispersity. For this reason, the produced magnesium oxide and/or magnesium oxide solid solution having an Mg partially substituted with M²⁺ may be excellent in adhesion and reactivity. According to the invention, taking advantage of such features, the highly-oriented composite metal salt may be utilized in the products such as anneal separating agents for electromagnetic steel plates, heat dissipation materials for resins and rubbers, acid acceptors for halogen-containing rubber, and vulcanization promoters for rubber.

### <Production Method>

### Step 1 (Coprecipitation Reaction)

(A) A mixed aqueous solution of a water-soluble magnesium salt and an organic ligand, or a mixed solution of a water-soluble magnesium salt, a water-soluble divalent metal (M²⁺) salt and an organic ligand is subjected to addition of 0.95 equivalent or lower, preferably 0.9 equivalent or lower, or particularly preferably 0.8 equivalent or lower and 0.6 equivalent or more of an alkali relative to the total equivalent of the divalent metal including the magnesium to obtain a coprecipitated product.

### Step 2 (Hydrothermal Treatment)

The coprecipitated product obtained in Step 1 is hydrothermally treated at 100°C or higher, preferably 120°C or higher, or particularly preferably from 180°C to 250°C, for 1 hour or more, and preferably for 2 to 10 hours. By changing the temperature of the hydrothermal treatment, a lateral width of a primary particle may be adjusted into the range of 0.21 to 40 µm.

### <Surface Treatment>

According to the invention, a variety of functions can be added to the highly-oriented composite metal salt through surface treatment. For example, in order to increase the compatibility with a resin and acid resistance, a substance such as selected from the following may be utilized: (a) higher fatty acids such as stearic acid and lauric acid; (b) alkali metal salts of said higher fatty acids; (c) anionic surfactants such as sodium dialkyl sulfosuccinate, alkyl ether sulfate, 2-ethylhexylalkyl/ sulfate ester/ sodium salt, sodium acylmethyl taurate, sodium alkylbenzene sulfonate and oleoylsarcosine; (d) phosphate esters such as acid forms, alkali metal salts or amine salts of monoesters or diesters of orthophosphoric acid and stearyl alcohol; (e) silane-based coupling agents such as vinylethoxysilane and γ-aminopropyltrimethoxysilane; (f) titanate-based coupling agents such as isopropyl triisostearoyl titanate; (g) aluminum-based coupling agents such as acetoalkoxyaluminum diisopropylate; (h) fatty acid esters of polyhydric alcohols such as sorbitan monostearate; (i) polycarboxylic acids such as sodium polyacrylate and sodium polystyrene sulfonate; and (j) alkali metal salts of polysulfonic acids. **In** order to increase acid resistance, coating such as silica coating by chemical adsorption of waterglass and subsequent addition of an acid; silica coating by hydrolysis of methyl silicate, ethyl silicate etc.; or silicone coating by silicone oil; may be made. **In** order to strengthen UV absorption and/or scattering, coating by fine particles of titanium oxide, zinc oxide, cerium oxide or the like may be made. A pearl pigment may be produced through the uniform surface treatment by adding fine particles of a metal oxide such as titanium oxide, iron oxide or zinc oxide to a dispersion of acid-resistance-improvement-surface-treated or untreated highly-oriented composite metal salt in a solvent such as water. Examples of a surface treatment agent as a paper flame retardant include carboxymethyl cellulose and sodium alginate.

The surface treatment may be preferably carried out in wet or dry manner. In a wet surface treatment, a surface treatment agent is added to a stirred dispersion of a composite metal salt in a solvent such as water or alcohol. In a dry surface treatment, a surface treatment agent is added to stirred highly-oriented composite metal salt powder in a high-speed stirrer such as a Henschel mixer. The amount of the surface treatment agent is appropriately selected and determined depending on a purpose. In general, the amount of the surface treatment agent may be preferably in the range of from 0.5 to 20% by weight relative to the weight of the highly-oriented composite metal salt.

### <Resin Composition>

**In** the resin composition in accordance with the invention, 0.01 to 300 parts by weight, preferably 0.5 to 200 parts by weight, and particularly preferably 1 to 100 parts by weight of highly-oriented composite metal salt may be mixed with 100 parts by weight of resin. The optimal amount of the highly-oriented composite metal salt to be mixed differs depending on a purpose. For example, the highly-oriented composite metal salt is mixed in an amount of from 1 to 40 parts by weight for the purpose of increasing the mechanical strength such as flexural modulus, bending strength or Izod strength of a resin; in an amount of from 1 to 20 parts by weight as an acid acceptor for a halogen-containing rubber; in an amount of from 50 to 200 parts by weight as a flame retardant or a gas-barrier agent of a resin; and in an amount of from 0.01 to 5 parts by weight as an acid acceptor for a resin such as polyamide.

### <Processing>

The method for mixing or kneading with a resin is not particularly limited, and may be any method as long as both materials can be uniformly mixed. For example, mixing or kneading may be carried out by using, for example, a single-screw or twin-screw extruder, an open roll, or a Banbury mixer. The molding method is not particularly limited either. Any known molding means may be employed, depending on a type of resin or rubber, or a desired type of molded product. Examples of the molding include injection molding, rotation molding, calendar molding, sheet-forming molding, transfer molding, lamination molding, and vacuum molding.

### <Types of Resins>

The resin to be used in the invention means a resin and/or rubber. Examples of the resin include: (A) thermoplastic resins such as polyethylene, copolymers of ethylene and the other α-olefins, copolymers of ethylene and vinyl acetate, ethyl acrylate or methyl acrylate, polybutene-1, poly(4-methylpentene-1), polystyrene, copolymers of styrene and acrylonitrile, copolymers of ethylene and propylene diene rubber, copolymers of ethylene and butadiene, polyvinyl acetate, polyvinyl alcohol, polyacrylate, polymethacrylate, polyurethane, polyester, polyether, polyamide, ABS, polycarbonate and polyphenylene sulfide; (B) thermosetting resins such as phenol resin, melamine resin, epoxy resin, unsaturated polyester resin and alkyd resin; and (C) rubbers such as EPDM, SBR, NBR, butyl rubber, chloroprene rubber, isoprene rubber, chlorosulfonated polyethylene rubber, silicon rubber, fluorine rubber, chlorinated butyl rubber, epichlorohydrin rubber and chlorinated polyethylene rubber.

Preferable examples of the resin include polypropylene, mixtures of polypropylene and olefin-based rubber, polyethylene, polyamide, EPDM, butyl rubber, and chloroprene rubber.

According to the invention, the resin composition may comprise, in addition to the highly-oriented composite metal salt, an optional conventional reinforcing agent such as talc, mica, glass fiber, and basic magnesium sulfate fiber. The amount of the reinforcing agent is from 1 to 50 parts by weight and preferably from 1 to 20 parts by weight relative to 100 parts by weight of the resin.

The resin composition may comprise, other than the optional reinforcing agent, a commonly used optional additive, for example, one or more appropriately selected from the group consisting of antioxidants; UV absorbers; lubricants; pigments such as carbon black; bromine-based or phosphate ester-based flame retardants; flame-retardant promoters such as zinc stannate, alkali metal stannate salt and carbon powder; and fillers such as calcium carbonate, zeolite and kaolin.

Hereinafter, the invention will be described in further detail based on Examples. However, it should not be construed that the invention is limited to or by them.

In Examples, (A) pre-treatment for XRD measurement, (B) measurement of primary particle size and (C) measurement of average secondary particle size of the highly-oriented composite metal salt were conducted by the methods described below.

### (A) XRD Measurement

The dried product which had passed through a 60-mesh sieve after having passed through a 30-mesh sieve was used as the sample powder.

### (B) Primary Particle Size

The above-mentioned powder which had passed through the 60-mesh sieve underwent ultrasonic dispersion treatment in water for 5 minutes, and then the maximum lateral width and the thickness of five primary particles were measured with a scanning electron microscope (SEM), and expressed as the arithmetic average values thereof.

### (C) Average Secondary Particle Size

Samples having undergone the ultrasonic dispersion treatment in the same manner as in (B) were measured by the laser diffraction method using a particle size distribution measuring device (LA 960 produced by HORIBA, Ltd.), where the 50% cumulative secondary particle size was regarded as the average secondary particle size.

The organic ligand was measured in accordance with absorptiometry.
i) In the case that the organic ligand is lactic acid, glycolic acid, triethanolamine or p-toluidine-2-sulfonic acid, the organic ligand was analyzed in accordance with the method described in the following article: L. N. Borshchevskaya etc., J. Analytical Chemistry, 71, No.8, 755-758 (2016).
ii) In the case that the organic ligand is ethylenediamine, the organic ligand was analyzed in accordance with the method described in the following article: Goro Hihara et al., Bull. Chem. Soc. Jpn, 54, 268-271 (1981).

The TG-DTA was measured with the TG-8120 produced by Rigaku Corporation under the conditions (atmosphere: air, and temperature increase rate: 20°C/min).

### Example 1

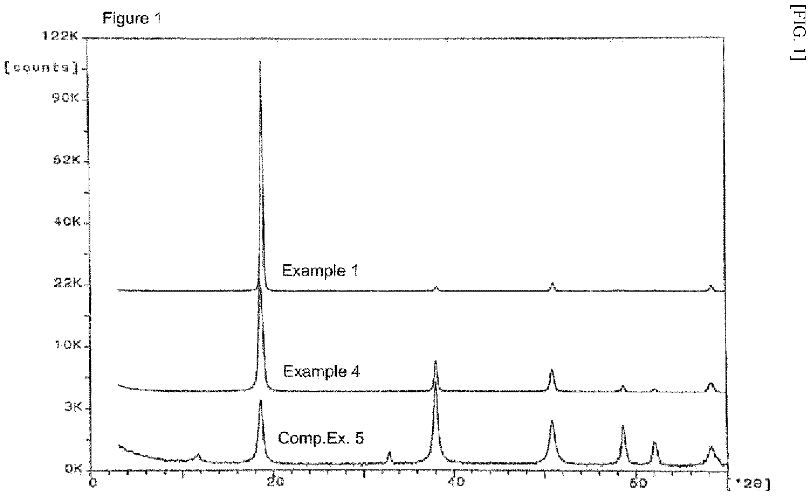

A mixed aqueous solution was prepared by dissolving 1 g of sodium lactate (reagent, 70% solution), equivalent to 1 mol% of organic ligand relative to Mg, in 400 mL of a 2.0 mol/L magnesium chloride (Reagent Grade 1) aqueous solution. The mixed aqueous solution (about 20°C) was placed in a 1L vessel, and subjected, with stirring, to addition of 320 mL of a 4 mol/L sodium hydroxide aqueous solution (about 20°C), where the sodium hydroxide corresponds to a 0.8 equivalent relative to Mg, to obtain a coprecipitated product. The coprecipitated product was transferred to a 1L autoclave, and hydrothermally treated at 200°C for 4 hours. The hydrothermally treated product was filtrated, washed with water, dried, and allowed to pass through a 60-mesh sieve after passing through a 30-mesh sieve. The XRD (powder X-ray diffraction method; Fig. 1) of the sample having passed through the 60-mesh sieve was measured, and the diffraction intensity ratio H (%) of the (101) plane to the (001) plane was measured.

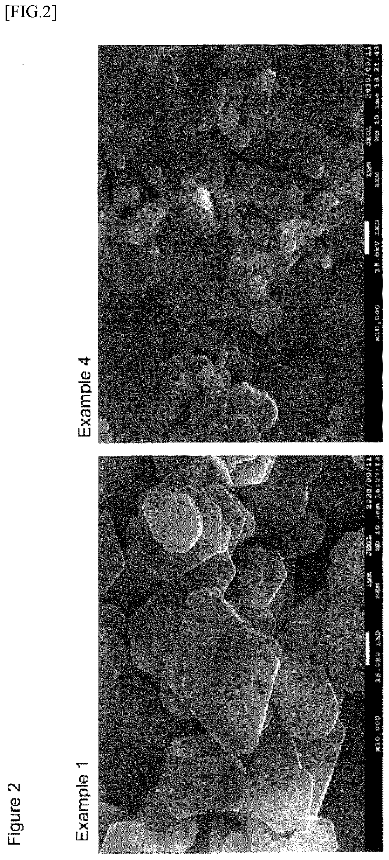

The sample having passed through the 60-mesh sieve was ultrasonically treated in an aqueous medium for 5 minutes, and subjected to the SEM analysis (Fig. 2) to measure the maximum lateral width and the thickness of five primary particles, where their average values thereof were obtained. On the other hand, the lactic acid content and Mg content were respectively measured by absorptiometry and the chelatometric titration after dissolving the sample in hydrochloric acid. In the absorptiometry, the pH was adjusted to about 3.1 by subjecting the sample solution containing 10 to 200 ppm of lactic acid to addition of a 0.2% aqueous ferric chloride solution in the same amount as that of the sample solution to develop yellow color, and then the intensity was measured at the wavelength of 390 nm, while using, as a control, the solution obtained by diluting a 0.2% ferric chloride aqueous solution with the same amount of water. The results are shown below in Table 1. The sample had the same XRD pattern as that of magnesium hydroxide, and the same Cd(OH)₂ type crystal structure as that of magnesium hydroxide. The interplanar distance (d=4.67 Å) of the (001) plane was shorter than that (d=4.77 Å) of magnesium hydroxide (POWDER DIFFRACTION FILE 7-239), so that the results of the XRD pattern support that the OH group of the magnesium hydroxide was partially substituted with the lactic acid ion as a solid solution.

The orientation H of the sample having passed through a 60-mesh sieve was 1%. The orientation of the product in accordance with the invention is evidently very high because the orientation of the aforementioned FILE 7-239 was calculated to be 111%.

The average primary particle size was 6.1 µm, and the average secondary particle size was 6.2 µm, which indicates that the secondary aggregation was nearly non-existent.

The endothermic decomposition peak temperature was 419°C based on TG-DTA data.

### Example 2

A composite metal salt was produced in the same manner as in Example 1 except that an amount of the aqueous sodium lactate solution was changed to 2.5 mol% relative to Mg. The measured results are shown in Table 1 below.

The XRD pattern was the same as that of magnesium hydroxide.

### Example 3

A composite metal salt was produced in the same manner as in Example 1 except that an amount of the aqueous sodium lactate solution was changed to 0.5 mol% relative to Mg. The measured results are shown in Table 1 below.

The XRD pattern was the same as that of magnesium hydroxide.

### [Comparative Example 1]

A composite metal salt was produced in the same manner as in Example 1 except that an amount of the aqueous sodium lactate solution was changed to 10 mol% relative to Mg. The measured results are shown in Table 1 below.

### [Comparative Example 2]

A composite metal salt was produced in the same manner as in Example 1 except that an amount of the 4 mol/L sodium hydroxide aqueous solution was changed to 400 mL, where the sodium hydroxide corresponds to a 1.0 equivalent relative to Mg. The measured results are shown in Table 1 below.

### [Comparative Example 3]

A composite metal salt was produced in the same manner as in Example 1 except that no sodium lactate was added. The measured results are shown in Table 1 below.

The endothermic decomposition peak temperature was 411°C based on TG-DTA data.

### [Comparative Example 4]

A composite metal salt was produced in the same manner as in Example 1 except that 150 mol% of sodium acetate relative to Mg was added instead of the sodium lactate, which was an organic ligand, in accordance with the conventional method for producing a high-aspect-ratio magnesium hydroxide (Example 1 of Patent Document 3). The measured results are shown in Table 1 below.

### Example 4

A composite metal salt was produced in the same manner as in Example 1 except that the temperature of hydrothermal treatment was changed from 200°C to 120°C. The measured results are shown in Table 1 below. The XRD and SEM photographs are shown in Figs. 1 and 2, respectively.

The XRD pattern was the same as that of magnesium hydroxide.

### [Comparative Example 5]

A composite metal salt was produced in the same manner as in Example 4 except that an amount of the 4 mol/L sodium hydroxide aqueous solution was changed to 400 mL, where the sodium hydroxide corresponds to a 1.0 equivalent relative to Mg (corresponding to Example 3 of Patent Document 4). The measured results are shown in Table 1 below.

The XRD is shown in Fig. 1.

### Example 5

A composite metal salt was produced in the same manner as in Example 1 except that 0.5 mol% of reagent ethylenediamine relative to Mg was used instead of the sodium lactate. The content of ethylenediamine was measured by absorptiometry after dissolving the sample in hydrochloric acid,. The measured results are shown in Table 1 below.

The XRD pattern was the same as that of magnesium hydroxide.

The endothermic decomposition peak temperature was 406°C based on TG-DTA data.

### Example 6

A composite metal salt was produced in the same manner as in Example 1 except that 1.5 mol% of reagent glycolic acid relative to Mg was added instead of the sodium lactate. The content of glycolic acid was measured by the same absorptiometry as in Example 1. The measured results are shown in Table 1 below.

The XRD pattern was the same as that of magnesium hydroxide.

The endothermic decomposition peak temperature was 428°C based on TG-DTA data.

### Example 7

A composite metal salt was produced in the same manner as in Example 1 except that 1 mol% of reagent glycine relative to Mg was added instead of the sodium lactate. The content of the glycine was measured by absorptiometry after dissolving the sample in hydrochloric acid. The measured results are shown in Table 1 below.

The XRD pattern was the same as that of magnesium hydroxide.

### Example 8

A composite metal salt was produced in the same manner as in Example 1 except that 4 mol% of reagent triethanolamine relative to Mg was added instead of the sodium lactate. The measured results are shown in Table 1 below. On the other hand, the content of the triethanolamine was measured by the same absorptiometry as in Example 5.

The XRD pattern was the same as that of magnesium hydroxide.

The endothermic decomposition peak temperature was 416°C based on TG-DTA data.

### Example 9

A composite metal salt was produced in the same manner as in Example 1 except that 2 mol% of reagent zinc chloride relative to Mg was mixed with the aqueous magnesium chloride solution, and the temperature of the hydrothermal treatment was changed to 250°C. The measured results are shown in Table 1 below.

The XRD pattern was the same as that of magnesium hydroxide.

### Example 10

A composite metal salt was produced in the same manner as in Example 1 except that 2 mol% of reagent nickel chloride relative to Mg was mixed with the aqueous magnesium chloride solution, and 2 mol% of glycolic acid relative to Mg was added instead of the sodium lactate. The measured results are shown in Table 1 below.

The XRD pattern was the same as that of magnesium hydroxide.

### Example 11

A composite metal salt was produced in the same manner as in Example 1 except that industrial magnesium chloride (special grade; produced by NAIKAI Salt Industries CO., LTD) was used instead of magnesium chloride (Reagent Grade 1), and the temperature of the hydrothermal treatment was changed to 250°C. The measured results are shown in Table 1 below.

The XRD pattern was the same as that of magnesium hydroxide.

### Example 12

A composite metal salt was produced in the same manner as in Example 11 except that reagent p-toluidine-2-sulfonic acid was used instead of the sodium lactate. The analysis of p-toluidine-2-sulphonic acid was performed by the HPLC method. The measured results are shown in Table 1 below.

The XRD pattern was the same as that of magnesium hydroxide.

The endothermic decomposition peak temperature was 405°C based on TG-DTA data.

### Example 13

A composite metal salt was produced in the same manner as in Example 1 except that 0.05 mol of industrial ferrous chloride solution (concentration of 32% by weight, produced by TAIKI CHEMICAL INDUSTRIES CO., Ltd.) relative to 1 mol of magnesium chloride was added, and lactic acid (Reagent Grade 1, 90% solution) was used instead of the sodium lactate. The measured results are shown in Table 1 below.

The color of the obtained product was white. The XRD pattern was the same as that of magnesium hydroxide. It was evident that divalent iron was dissolved as a solid solution. After dissolving the sample in hydrochloric acid and then subjecting the dissolved sample to the chelatometric titration, the composition of this solid solution was found to be Mg_{0.95}Fe²⁺_{0.05}(OH)₂.

The obtained product can be added to resin film for foodstuff packaging as an oxygen absorbent, taking advantage of the fact that divalent iron is converted into a stable trivalent iron through oxidation with oxygen. Furthermore, since this solid solution can easily dissolve in stomach acid to supply an easily absorbable divalent iron, it may also be used as a therapeutic agent for iron-deficiency anemia for humans. Because existing iron agents have a metallic taste and easily generates a rejection response, there are quite many people who have found it difficult to continuously use the existing iron agents in a required period of one month or more. On the other hand, this solid solution is substantially the same as magnesium hydroxide so that there is no metallic taste and it is easy to take. Thus, this solid solution also brings an advantage that it can be taken as a medicine continuously in a long period of time.

**[Table 1]**

| | type of organic ligand or organic acid | amount of organic ligand or organic acid | content of "A" in formula (1) | orientation H | interplanar distance d of (001) plane | lateral width of primary particle | thickness of primary particle | aspect ratio |
|---|---|---|---|---|---|---|---|---|
| | | (mol%)*1 | (mol%)*2 | (%)*3 | (Å) | (µm) | (µm) | |
| Example1 | Na lactate | 1.0 | 1.03 | 1 | 4.67 | 6.1 | 45 | 135 |
| Example2 | Na lactate | 2.5 | 1.27 | 9 | 4.70 | 4.7 | 52 | 90 |
| Example3 | Na lactate | 0.5 | 0.49 | 7 | 4.70 | 1.5 | 18 | 83 |
| Comp.Ex.1 | Na lactate | 10 | 1.34 | 44 | 4.70 | 0.91 | 28 | 32 |
| Comp.Ex.2 | Na lactate | 1.0 | 0.92 | 138 | 4.75 | 0.45 | 150 | 3 |
| Comp.Ex.3 | none | 0 | 0 | 70 | 4.78 | 1.1 | 210 | 5 |
| Comp.Ex.4 | Na acetate | 150 | 0 | 7 | 4.77 | 2.8 | 96 | 29 |
| Example4 | Na lactate | 1.0 | 1.01 | 19 | 4.73 | 0.47 | 17 | 28 |
| Comp.Ex.5 | Na lactate | 1.0 | 0.78 | 157 | 4.75 | 0.28 | 57 | 5 |
| Example5 | ethylenediamine | 0.5 | 0.03 | 4 | 4.77 | 5.2 | 48 | 108 |
| Example6 | glycolic acid | 1.5 | 1.1 | 2 | 4.73 | 3.0 | 46 | 65 |
| Example7 | glycine | 1.0 | 0.15 | 6 | 4.73 | 3.9 | 34 | 115 |
| Example8 | triethanolamine | 1.0 | 0.33 | 6 | 4.71 | 2.3 | 12 | 191 |
| Example9 | lactic acid | 2.0 | 1.2 | 4 | 4.73 | 3.8 | 42 | 90 |
| Example10 | glycolic acid | 2.0 | 1.3 | 1 | 4.73 | 3.2 | 29 | 110 |
| Example11 | Na lactate | 1.0 | 0.89 | 0.3 | 4.75 | 8.9 | 60 | 148 |
| Example12 | p-toluidine-2-sufonic acid | 1.0 | 0.04 | 2 | 4.77 | 3.8 | 44 | 86 |
| Example13 | lactic acid | 1.0 | 0.89 | 2 | 4.75 | 2.6 | 35 | 74 |

| | | | | | | | | |
|---|---|---|---|---|---|---|---|---|
| *1 It is mol% of the organic substance relative to 1 mol of Mg. *2 It is mol% of the organic ligand relative to 1 mol of Mg. *3 It is a ratio (%) of the X-ray diffraction intensity of the (101) plane to the X-ray diffraction intensity of the (001) plane. | | | | | | | | |

### Example 14

### <Resin Composition>

The 500 g powder of the highly-oriented composite metal salt produced by the method of Example 1 was added to 5 L of water, and dispersed with a Chemistirrer. Then it was heated to 80°C, and stirred while being subjected to addition of 10 g of sodium stearate for surface treatment. Then the resulting product was filtrated, washed with water, and dried. The 12 parts by weight (equivalent to about 20% by weight of the total) of the dried product was mixed with 100 parts by weight of polypropylene and 0.2 parts by weight of an antioxidant (IRGANOX 1010). The resulting mixture was kneaded by means of a twin-screw extruder at about 190°C to produce pellets. The pellets were vacuum dried, and then injection-molded at about 230°C with an injection molding machine to produce test pieces. The flexural modulus and Izod impact strength were measured using these test pieces in accordance with JIS 7171 and JIS 7110, respectively. The melt flow index (MFR) was measured using the pellets in accordance with JIS 7210. The results thereof are shown in Table 2 below.

### [Comparative Example 6]

A resin composition was produced in the same manner as in Example 14 except that the high-aspect-ratio magnesium hydroxide produced by the method (Patent Document 3) of Comparative Example 4 was used instead of the highly-oriented composite metal salt produced by the method of Example 1. Evaluation results are shown in Table 2 below.

### [Comparative Example 7]

A resin composition was produced in the same manner as in Example 14 except that talc (primary particle size of 5 µm, thickness of 0.2 µm, and aspect ratio of 20), which was often used as a resin-reinforcing agent for automobiles, was used instead of the highly-oriented composite metal salt. In Reference Example, a resin composition was produced in the same manner as in Example 14 except that no reinforcing agent was used. Evaluation results are shown in Table 2 below.

**[Table 2]**

| | type of reinforcing agent | flexural modulus (MPa) | Izod impact strength at 23°C (KJ/m²) | MFR (g/10mins) |
|---|---|---|---|---|
| Example14 | highly-oriented composite metal salt | 3,020 | 7.2 | 38 |
| Comp.Ex.6 | conventional high-aspect-ratio magnesium oxide | 2,450 | 6.4 | 33 |
| Comp.Ex.7 | talc | 1,860 | 4.1 | 29 |
| Reference Ex. | none | 1,200 | 6.0 | 32 |

### Example 15

### <Cosmetic material with superior slipperiness (lubricity)>

Using the highly-oriented composite metal salt powder having passed through a 60-mesh sieve in Example 11 in accordance with the invention, the static friction coefficient and dynamic friction coefficient, which correspond to the spreading performance as a cosmetic material, were measured. The results are shown in Table 3 below. It is evident that the highly-oriented composite metal salt powder in accordance with the invention has slipperiness (lubricity) as good as or better than existing lubricated cosmetic materials. The filter cake after the hydrothermal treatment in the production of this powder showed a pearl tone high gloss.

### [Comparative Examples 8-1 and 8-2]

The static friction coefficient and dynamic friction coefficient were measured by using commercially available mica (SERICITE JS-1) in Comparative Example 8-1 and by using commercially available talc (JA-467 talc) in Comparative Example 8-2, where the mica and the talc were typical lubricant powders for cosmetic material. The results thereof are shown in Table 3 below.

The static friction coefficient and dynamic friction coefficient were measured with the following device under the following conditions.
Device: Static-dynamic friction measuring device TL201Tt produced by Trinity-Lab Inc.
Test pieces: 0.5 mg/m² of test powder (having passed through a 60-mesh sieve) applied on a 5 cm x 10 cm sheet
Load: 25 g
Rate of sample movement: 1 mm/sec
Measured distance range: 20 mm

**[Table 3]**

| | type of lubricant powder | static friction coefficient | dynamic friction coefficient |
|---|---|---|---|
| Example15 | invented product | 0.79 | 0.46 |
| Comp.Ex.8-1 | mica | 0.84 | 0.48 |
| Comp.Ex.8-2 | talc | 0.84 | 0.62 |

It is known that the slipperiness (lubricity) of cosmetics becomes better as the dynamic friction coefficient becomes smaller. Accordingly, it is evident that the slipperiness of the invented product in Example 15 is as good as, or better than those of the commercially available mica and talc in Comparative Examples 8-1 and 8-2.

## Claims

1. A highly-oriented composite metal salt having a hexagonal Cd(OH)₂ type crystal structure and an X-ray diffraction intensity ratio (orientation H) of the (101) plane to the (001) plane of 60% or less, and being denoted by the following formula (1):
(Mg)₁₋ₓ(M²⁺)ₓ(OH)_{2-ny}A_{y} (1),
wherein "M²⁺" denotes at least one divalent metal other than Mg; "A" denotes at least one organic ligand; "x" denotes a number in a range of 0 ≤ x < 0.2, preferably 0 ≤ x < 0.1 and particularly preferably 0.01 ≤ x < 0.06; "y" denotes a number in a range of 0 < y < 0.05, preferably 0.0001 < y <0.02 and particularly preferably 0.001 < y <0.015; and "n" denotes an integer of from 1 to 4, preferably 1, or zero.

2. The highly-oriented composite metal salt according to claim 1, wherein the X-ray diffraction intensity ratio is 60% or less and the "n" denotes an integer of from 1 to 4.

3. The highly-oriented composite metal salt according to claim 1, wherein the X-ray diffraction intensity ratio is 30% or less, more preferably 3% or less and particularly preferably 1% or less, and the "n" denotes an integer of from 1 to 4.

4. The highly-oriented composite metal salt according to claim 1, wherein the X-ray diffraction intensity ratio is 60% or less and more than 30%, and the "n" denotes an integer of from 1 to 4.

5. The highly-oriented composite metal salt according to claim 1, wherein the "n" denotes zero.

6. The highly-oriented composite metal salt according to any one of claims 1 to 5, wherein an average lateral width of primary particles of the highly-oriented composite metal salt is from 0.4 to 50 µm.

7. The highly-oriented composite metal salt according to any one of claims 1 to 6, wherein the X-ray diffraction intensity ratio (orientation H) is 6% or less, and an average lateral width of primary particles of the highly-oriented composite metal salt is from 2 to 50 µm.

8. The highly-oriented composite metal salt according to any one of claims 1 to 7, wherein the "A" in the formula (1) is at least one organic ligand selected from the group consisting of oxycarboxylic acids, amines, amino acids, polyhydric alcohols and polyphenols.

9. The highly-oriented composite metal salt according to any one of claims 1 to 8, wherein the "M²⁺" in the formula (1) is at least one divalent metal selected from the group consisting of Ca²⁺, Mn²⁺, Fe²⁺, Co²⁺, Ni²⁺, Cu²⁺ and Zn²⁺.

10. The highly-oriented composite metal salt according to any one of claims 1 to 9, wherein the "x" in the formula (1) denotes a number in a range of 0.001 ≤ x < 0.02.

11. The highly-oriented composite metal salt according to any one of claims 1 to 10, wherein the highly-oriented composite metal salt is surface-treated with at least one selected from the group consisting of higher fatty acids, alkali metal salts, anionic surfactants, phosphoric acid esters (phosphates), silane-based coupling agents, titanate-based coupling agents, aluminate-based coupling agents, fatty acid esters, polycarboxylic acids, alkali metal salts, waterglass, methyl silicate, ethyl silicate, silicone oil, titanium oxide, zinc oxide, cerium oxide, fine particles of a metal oxide, carboxymethyl cellulose and sodium alginate.

12. A resin composition comprising:
100 parts by weight of a resin, and
0.01 to 300 parts by weight of the highly-oriented composite metal salt according to any one of claims 1 to 11.

13. A method for producing a highly-oriented composite metal salt of any one of claims 1 to 12, the method comprising steps of:
subjecting an aqueous solution of one or more divalent metals to addition of less than 10% by mol of an organic ligand relative to a total molar amount of the divalent metals to obtain a mixture, wherein the aqueous solution of one or more divalent metals comprises magnesium;
reacting the mixture with the 0.95 equivalent or less of an alkali relative to a total equivalent of the divalent metals to obtain a coprecipitated product; and
hydrothermally treating the coprecipitated product at 100°C or higher.

14. The method according to claim 13, wherein the at least one divalent metal other than Mg is at least one selected from the group consisting of Ca²⁺, Mn²⁺, Fe²⁺, Co²⁺, Ni²⁺, Cu²⁺ and Zn²⁺.
